# EUROPEAN PATENT APPLICATION

(11) **EP 2 336 774 A1**
(43) Date of publication of application: **22.06.2011**
(21) Application number: 08800606.9
(22) Date of filing: 16.09.2008
(51) Int. Cl.: G01N 33/53, G01N 33/543, G01N 33/558, G01N 33/535

(54) **TWO USES FLUID TEST CHIP**

(71) Applicant: Actherm Inc., Hsinchu 30078 (TW)
(72) Inventor: HSIEH, Chih-Wei, Zhubei Hsinchu County 302 (TW); HSIEH, Wen-Pin, Sanwan Shiang Miaoli County 352 (TW); WU, Yi-Jen, Hsinchu City 30078 (TW)
(74) Representative: Lang, Christian
(86) International application number: PCT/CN2008/001613
(87) International publication number: WO 2010/031201

(57) **Abstract**

A combinatory testing strip including a substrate is disclosed. A first channel for a biochemical assay and a second channel for an immunological assay are provided concavely on an upper surface of the substrate. The results of both assays are detected by a sensor. Each channel includes a first area for receiving a fluid sample, a second area for delivering the fluid sample and a third area where the fluid sample reacts. These three areas are connected successively. A nitrocellulose layer having a hollow-matrix conformation is formed at a bottom of each of the second and third areas of both channels. Each of the nitrocellulose layers of the second areas comprises an average thickness that is not greater than that of each the nitrocellulose layers of the third areas. A reaction material is formed in the hollow-matrix conformation. The third areas of the first and second channels are both located on a line conforming a relative motion path of the sensor and the combinatory testing strip.

## Description

### BACKGROUND OF THE INVENTION

### 1. Technical Field

The present invention relates to testing strips, and more particularly, to a testing strip for biological and immunological assays.

### 2. Description of Related Art

Testing strips are conventionally used in biochemical tests and immunological tests. A typical testing strip includes a substrate or base formed with channels or micro-channels and processed with hydrophilic/hydrophobic surface treatment. Since the channels are bordered by non-absorbent material, and fluid samples to be tested are usually viscous compositions containing, for example, protein or carbohydrate, a fluid sample flowing in the channels tends to adhere to surfaces of the channels and cannot be fully reacted. Consequently, the fluid sample is wasted, if not leading to errors in test results.

In addition, the conventional testing strips are provided with micro-channels to facilitate fluid delivery, in which the micro-channels cause a capillary action that draws a fluid sample through the channels to a reaction/detection region. Alternatively, a fluid sample may be introduced into the channels with a driving force provided by, for example, a pressurizing means or a vacuum- or negative pressure-generating means, thereby propelling the fluid sample through the channels. Another approach to promoting fluid delivery is to provide the channels with one or more micro-actuators or micro-valves through which a fluid sample will pass successively before arriving at a reaction/detecting region. However, in any of the aforesaid approaches, air bubbles of various sizes tend to be generated in, or entrained into, fluid samples to be tested after the samples are introduced into the channels. These bubbles, when causing channel blockage, may result in test errors or even test failure. Moreover, installation of the micro-actuators or micro-valves will add to the overall difficulty of design and the cost of testing strips.

Besides, during manufacture of the conventional testing strips, the channels or microfluidic-channels are usually formed on the substrates by micro-injection forming or imprinting, using expensive die making process such as micro-machining or LIGA (abbreviation of "Lithographie GalVanoformung Abformung", or "Lithography Electroforming Micro Molding" in english) which, coupled with early wear and tear of molds, increases the total cost incurred in making testing strips.

Furthermore, it is understood that the reaction materials or reagents varies from the categories of assays. However, the conventional testing strip typically comprises the reaction materials that are applicable only to one category of assay. Thus, multiple assays can be performed on the same fluid sample only with more than one testing strip, which, however, render testing inconvenient and time-consuming.

### BRIEF SUMMARY OF THE INVENTION

In order to overcome the abovementioned shortcomings, the present invention provides a combinatory testing strip that can perform a biochemical assay and a immunological assay simultaneously and be detected by a sensor. The combinatory testing strip essentially comprises a substrate having an upper surface concavely provided with a first channel for a biochemical assay and a second channel for an immunological assay. Each of the first and second channels includes a first area for receiving a fluid sample, a second area for delivering the fluid sample and a third area where the fluid sample reacts. These three areas are connected successively. The combinatory testing strip is characterized in that a nitrocellulose layer having a hollow-matrix conformation is formed at each bottom of the second and third areas of the two channels. In addition, each of the nitrocellulose layers of the second areas comprises an average thickness that is not greater than the average thickness of the nitrocellulose layers of the third areas. A reaction material is formed in the hollow matrices of the nitrocellulose layers. Besides, the third areas of the first and second channels are both located on a line conforming a relative motion path of the sensor and the combinatory testing strip.

Hence, a primary object of the present invention is to provide a combinatory testing strip capable of simultaneously performing a biochemical assay and an immunological assay on a fluid sample.

Another object of the present invention is to provide a combinatory testing strip that has thin absorptive nitrocellulose layers at the bottom of the channel. The thin absorptive nitrocellulose layers act as sample delivering and/or separating function. The channel thus has lower residual of samples in contrast to the traditional microfluidic channel, and low volume of samples needed for multi-analytes detection in a test is realized.

Still another object of the present invention is to provide a combinatory testing strip that comprises absorptive nitrocellulose layers having a constant volumetric absorptive capacity and thus allows a quantitative assay to be conducted via controlling the volume of the nitrocellulose layers.

A further object of the present invention is to provide a combinatory testing strip formed with nitrocellulose layers with a hollow-matrix configuration, which is capable of destroying and eliminating the air bubbles in the fluid sample when the fluid sample flows through the hollow matrix, as well as preventing the bubbles from blocking the channel or the microfluidic channel of the substrate. Thus, an accurate result of the quantitative assay could be assured.

### BRIEF DESCRIPTION OF THE SEVERAL VIEWS OF THE DRAWINGS

The invention as well as a preferred mode of use, further objectives and advantages thereof will be best understood by referring to the following detailed description of an illustrative embodiment in conjunction with the accompanying drawings, wherein:
Fig. 1 is a schematic perspective view of a combinatory testing strip according to a preferred embodiment of the present invention;
Fig. 2 is a top view of the combinatory testing strip according to the preferred embodiment of the present invention;
Fig. 3 is a schematic sectional view of a first channel of the combinatory testing strip according to the preferred embodiment of the present invention; and
Fig. 4 is a schematic sectional view of a second channel of the combinatory testing strip according to the preferred embodiment of the present invention.

### DETAILED DESCRIPTION OF THE INVENTION

The present invention discloses a combinatory testing strip in which the physical and chemical principles as well as solution casting techniques involved are well known to those skilled in the art. Therefore, a detailed description of such principles and techniques is omitted herein for brevity. Besides, the drawings referred to in the following description are not drawn to actual scale and need not be so because they are intended to demonstrate features of the present invention only schematically.

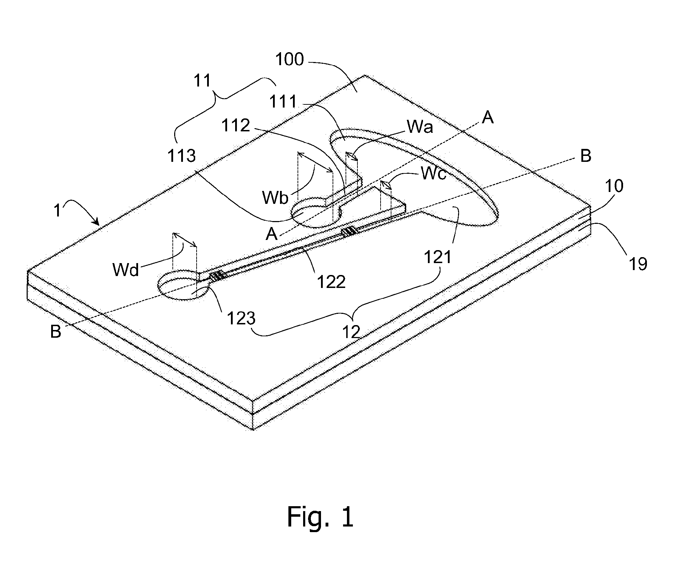

Please refer to Fig. 1 for a preferred embodiment of the present invention. The combinatory testing strip 1 is applicable to perform simultaneously a biochemical assay and an immunological assay. As shown in the drawing, the combinatory testing strip 1 essentially comprises a substrate 10 and a supporter 19. The substrate 10 has an upper surface 100 concavely provided with a first channel 11 for the biochemical assay and a second channel 12 for the immunological assay. The first channel 11 comprises a first area 111, a second area 112 and a third area 113. These three areas 111, 112, and 113 are successively connected. The second channel 12 comprises a first area 121, a second area 122 and a third area 123 and these three areas 121, 122, and 123 are also successively connected. The first area 111 of the first channel 11 and the first area 121 of the second channel 12 connect with each other for receiving a fluid sample. The fluid sample is introduced into the interconnected first areas 111 and 121 and then delivered separately by the second areas 112 and 122 to the third areas 113 and 123, respectively. When the fluid sample that flows in the first channel 11 reaches the third area 113, the analytes in the fluid sample will undergo biochemical reaction to generate a signal for detection. Similarly, when a portion of the fluid sample that flow in the second channel 12 arrives at the third area 123, the analytes in the fluid sample will undergo immunological reaction to generate another signal for detection. The substrate 10 is preferably made of a biocompatible material.

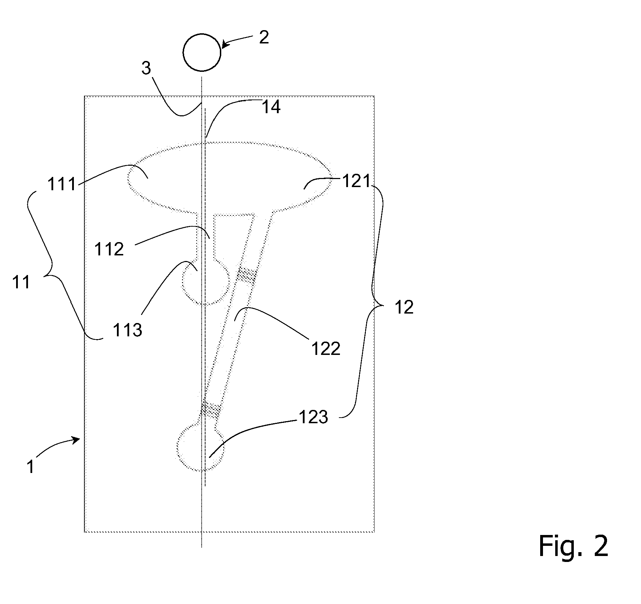

Fig. 2 shows a top view of the combinatory testing strip 1 of the present invention. To facilitate the detection of signals generated by the assays, the third area 113 of the first channel 11 and the third area 123 of the second channel 12 are both located on a line 14 that conforms a relative motion path 3, so that a sensor 2(for example, an optical sensor, isotope detector, or an electrode) only has to move along the relative motion path 3 to detect the signals generated from the third area 113 of the first channel 11 and the third area 123 of the second channel 12. The line 14 is preferable, but not limited to, a longitudinal axis of the substrate 10. The type of line 14 can be a curve, an arc, or any form conforming the relative motion path 3 that the sensor 2 move along. On the other hand, when the testing strip 1 is used with the reader with a fixed sensor, the line 14 can also be parallel to the relative motion path 3 along which the testing strip 1 is delivered into the reader. When detecting, it is preferable, but not necessary, that one of the sensor and the testing strip 1 moves whereas the other doesn't.

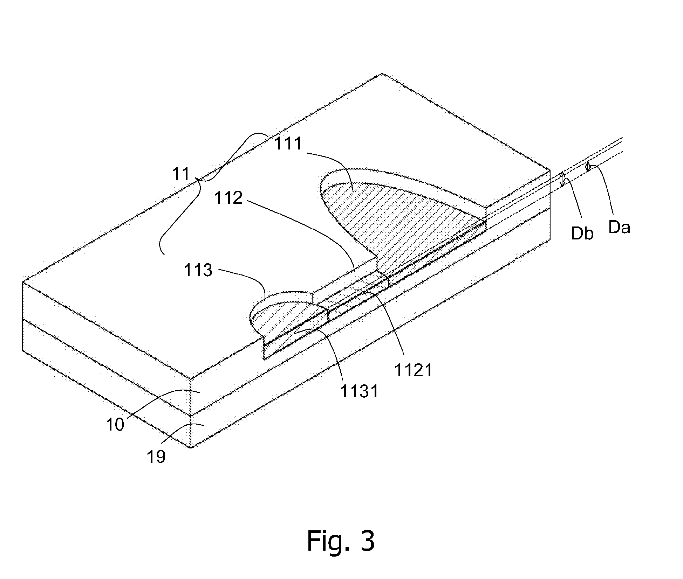

Referring now to Fig. 3, which is a sectional view of the first channel 11 taken along a line A-A in Fig. 1, the first channel 11 comprises the nitrocellulose layers 1121 and 1131 disposed at the bottoms of the second area 112 and the third area 113. Each of the nitrocellulose layers 1121 and 1131 has a hollow-matrix conformation. The nitrocellulose layer 1121 comprises an average thickness Da that is smaller than the average thickness Db of the nitrocellulose layer. In addition, a reaction material is formed in the hollow-matrix conformation of the nitrocellulose layers 1121 and 1131. The composition of the reaction material varies from the kind of analytes and the category of assays. Besides, the porous hollow-matrix conformation of the nitrocellulose layers 1121 and 1131 serves to absorb the fluid sample that comes from the first area 111, thereby allowing the analytes of the fluid sample to react with the reaction material contained in the nitrocellulose layer 1131.

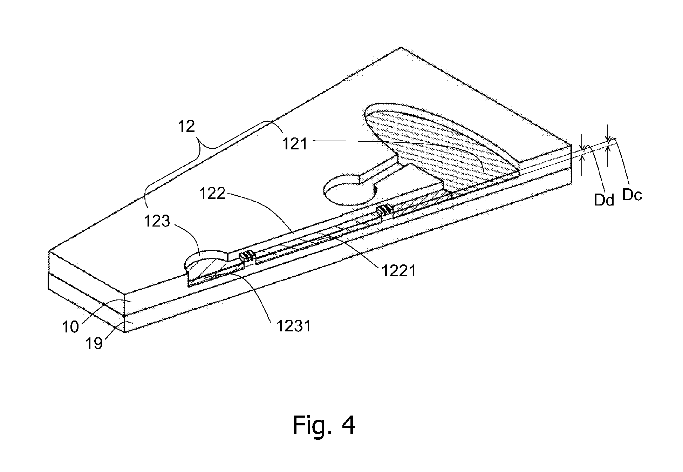

Please now refer to Fig. 4 for a sectional view of the second channel 12 taken along a line B-B in Fig. 1. The second channel 12 is similar to the first channel 11 in having nitrocellulose layers 1221 and 1231 formed respectively at bottoms of the second and third areas 122 and 123. Each of the nitrocellulose layers 1221 and 1231 has a hollow-matrix conformation. In addition, analogous to the nitrocellulose layers 1121 and 1131 of the first channel 11, hollow-matrix conformation of the nitrocellulose layers 1221 and 1231 also contain a reaction material and are porous so as to absorb the fluid sample that comes from the first area 121, thereby allowing the analytes of the fluid sample to react with the reaction material contained in the nitrocellulose layer 1231.

Since both of the first and second channels 11 and 12 are provided with the absorptive nitrocellulose layers 1121, 1131, 1221 and 1231, the fluid sample will not adhere to the surfaces of the first and second channels 11 and 12. Furthermore, when the fluid sample flows through the hollow-matrix conformations of the nitrocellulose layers 1121, 1131, 1221 and 1231, air bubbles in the fluid sample will be destroy and eliminated so as not to block the first and second channels 11 and 12.

Additionally, in order to reduce the influence of capillary effect excerted between the channels and the fluid sample, the configurations of the first and second channels 11 and 12 disclosed in the present invention are not similar to those of the conventional micro-channels. As shown in Fig. 1, the second area 112 of the first channel 11 has a width Wa, the third area 113 of the first channel 11 has a width Wb, the second area 122 of the second channel 12 has a width Wc and the third area 123 of the second channel 12 has a width Wd. All the widths Wa, Wb, Wc and Wd are preferably at least 0.3 mm.

The nitrocellulose layers 1121, 1131, 1221 and 1231 are formed in the following manner. To begin with, a nitrocellulose powder is mixed with an organic solvent containing esters and ketones to form a nitrocellulose solution. Then the nitrocellulose solution is poured onto the bottoms of the second and third areas 112 and 113 of the first channel 11 and the bottoms of the second and third areas 122 and 123 of the second channel 12 in a casting process. After drying, the nitrocellulose layer 1121 is formed at the bottom of the second area 112 of the first channel 11, the nitrocellulose layer 1131 is formed at the bottom of the third area 113 of the first channel 11, the nitrocellulose layer 1221 is formed at the bottom of the second area 122 of the second channel 12 and the nitrocellulose layer 1231 is formed at the bottom of the third area 123 of the second channel 12. For a better result of the casting process, the first and second channels 11 and 12 preferably have a surface roughness ranging from 3 µm to 50 µm.

Preferably, the nitrocellulose powder is mixed with the organic solvent containing esters and ketones at a volumetric ratio of 1: 9 for a hollow matrix with a better structure. Because each volumetric unit of nitrocellulose has a constant absorptive capacity, the required volume of the nitrocellulose solution can be derived from a desired volume of the fluid sample to be absorbed, before the casting process begins. As a result, the required volume of the fluid sample of the combinatory testing strip 1 will be fixedly set after the formation of the nitrocellulose layers 1121, 1131, 1221 and 1231, so that the resultant combinatory testing strip 1 is suitable for an assay in a small volume.

The reaction materials in the nitrocellulose layers 1121, 1131, 1221 and 1231 can be formed by the two following manners that one is to be formed in a readily formed nitrocellulose layers and the other is to be formed simultaneously with the nitrocellulose layers.

The reaction materials are formed in a readily formed nitrocellulose layers in the following manners. First, reaction solutions containing the reaction materials are injected into the nitrocellulose layers 1121, 1131, 1221 and 1231 which have already been readily formed. The reaction solutions are then dried in an air-drying process or a lyophilization process so that the reaction materials are left in the nitrocellulose layers 1121, 1131, 1221 and 1231 in the form of powder.

To form the reaction materials simultaneously with the nitrocellulose layers, the reaction solution containing the reaction materials is firstly mixed with the nitrocellulose solution comprising the nitrocellulose powder and the organic solvent containing esters and ketones, so that after an air-drying process or a lyophilization process, the nitrocellulose solution is thus dried to form the nitrocellulose layers 1121, 1131, 1221 and 1231 while the reaction materials are left therein in a powder form.

As mentioned previously, the first channel 11 is intended for a biochemical assay and the second channel 12 is intended for an immunological assay. To detect different analytes of the physiological fluid needs different assays, and different categories of assays require different kinds of reaction materials, which result in different categories of signals. Thus, the reaction material formed in the nitrocellulose layers 1121 and 1131 of the first channel 11 shall have a composition different from that of the reaction material formed in the nitrocellulose layers 1221 and 1231 of the second channel 12. A biochemical quantitative assay, for example, is usually carried out via the enzymatic reaction of the analytes in the biological fluid sample and a chemical luminating reagent, which is catalyzed by the suitable enzymes, to generate optical signals with specific wavelengths for detection. Accordingly, the reaction material in the nitrocellulose layers 1121 and 1131 of the first channel 11 will contain the enzyme and the corresponding chemical reagents required. On the other hand, when the scenario comes to the quantitative detection of a certain protein in the physiological fluid sample, such as α -fetoprotein, the testing assay usually utilize a antibody which can specifically recognize the targeted protein and other corresponding chemical reagents to generate detectable signals. Accordingly, the reaction material in the nitrocellulose layers 1221 and 1231 of the second channel 12 will contain the necessary antibody and the corresponding chemical reagent.

In addition, in a preferred mode of the present invention, the second channel 12 may further include a fourth area (not shown) whose bottom is also formed with a nitrocellulose layer for accommodating excess of the fluid sample. Besides, as shown in Fig. 4, the nitrocellulose layer 1221 comprises an average thickness that preferably equals to the average thickness Dd of the nitrocellulose layer 1231.

The above description is intended only to demonstrate the preferred embodiment of the present invention and not to limit the scope of the invention. Moreover, as the contents disclosed herein should be readily understood and can be implemented by those skilled in the art, all equivalent changes or modifications which do not depart from the spirit of the present invention should be encompassed by the appended claims.

## Claims

1. A combinatory testing strip for performing a biochemical assay and a immunoassay simultaneously and detecting by a sensor, comprising a substrate having an upper surface concavely provided with a first channel for a biochemical assay and a second channel for an immunological assay, and each of the first and second channels having a first area for receiving a fluid sample, a second area and a third area that are connected successively, wherein the combinatory testing strip is **characterized in that**:
at the bottom thereof, each of the second and the third area comprises a nitrocellulose layer having a hollow-matrix conformation, and the second area is for delivering the fluid sample and the third area is where the fluid sample reacts;
the nitrocellulose layer of the second area comprises an average thickness which is not greater than that of the nitrocellulose layer of the third area;
a reaction material is formed in the hollow-matrix conformation of the nitrocellulose layers; and
both the third areas of the first and second channels are located on a line conforming a relative motion path of the sensor and the combinatory testing strip.

2. The combinatory testing strip of Claim 1, wherein the first areas of the first and second channels connect with each other.

3. The combinatory testing strip of Claim 1, wherein the average thickness of the nitrocellulose layer of the second area is smaller than that of the nitrocellulose layer of the third area.

4. The combinatory testing strip of Claim 3, wherein the nitrocellulose layers are formed by casting a nitrocellulose solution onto the bottoms of the second and third areas followed by a drying process.

5. The combinatory testing strip of Claim 4, wherein the nitrocellulose solution is formed by mixing a nitrocellulose powder with a solvent containing esters and ketones.

6. The combinatory testing strip of Claim 4, wherein the nitrocellulose powder is mixed with the solvent containing esters and ketones at a volumetric ratio of 1: 9.

7. The combinatory testing strip of Claim 3, wherein each of the second and third areas has a width of at least 0.3 mm.

8. The combinatory testing strip of Claim 3, wherein the substrate is made of a biocompatible material.

9. The combinatory testing strip of Claim 3, wherein each of the first and second channels has a surface roughness ranging from 3 µm to 50 µm.

10. The combinatory testing strip of Claim 4, wherein the reaction material in the hollow-matrix conformation is in a powder form and formed by adding a reaction solution containing the reaction material into the nitrocellulose layers, followed by a drying process.

11. The combinatory testing strip of Claim 4, wherein the reaction material in the hollow-matrix conformation is in a powder form and formed by mixing a reaction solution containing the reaction material with the nitrocellulose solution and then casting onto the bottoms of the second and third areas of both the first and the second channels, followed by a drying process, so that the nitrocellulose solution forms the nitrocellulose layers while the reaction material is left in the nitrocellulose layers in a powder form.

12. The combinatory testing strip of Claim 3, wherein the reaction material comprises an enzyme and a chemical reagent.

13. The combinatory testing strip of Claim 3, wherein the reaction material comprises an antibody and a chemical reagent.

14. The combinatory testing strip of Claim 3, wherein the channel further comprises a fourth area having a nitrocellulose layer which is formed at the bottom thereof and also has a hollow-matrix conformation for accommodating excess of the fluid sample.

15. The combinatory testing strip of Claim 14, wherein the nitrocellulose layer of the second area of the second channel comprises an average thickness that equals to the average thickness of the nitrocellulose layer of the third area of the second channel.

16. The combinatory testing strip of Claim 3, wherein the nitrocellulose layer of the second area of the first channel comprises an average thickness that is smaller than the average thickness of the nitrocellulose layer of the third area of the first channel.
